# EUROPÄISCHE PATENTANMELDUNG

(11) **EP 1 132 374 A2**
(43) Veröffentlichungstag der Anmeldung: **12.09.2001**
(21) Anmeldenummer: 01105000.2
(22) Anmeldetag: 01.03.2001
(51) Int. Cl.: C07C 217/90, C08G 73/22, C07C 205/38, C07D 471/04, C08G 73/10

(54) **Bis-o-aminophenole und o-Aminophenolcarbonsäuren**

(30) Priorität: 10.03.2000 DE 10011608
(71) Anmelder: Infineon Technologies AG, 81669 München (DE)
(72) Erfinder: Hausmann, Jörg, 91056 Erlangen (DE); Maier, Gerhard, 80807 München (DE); Sezi, Recai, 91341 Roettenbach (DE)
(74) Vertreter: MÜLLER & HOFFMANN Patentanwälte

(57) **Zusammenfassung**

Die Erfindung betrifft neue Bis-o-aminophenole und o-Aminophenolcarbonsäuren folgender Struktur: 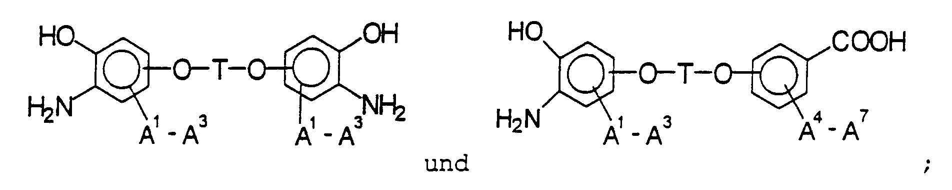 dabei gilt Folgendes:
A¹ bis A⁷ sind - unabhängig voneinander - H, F, CH₃, CF₃, OCH₃ oder OCF₃;
T ist ein aromatischer oder heterocyclischer Rest.

## Beschreibung

Die Erfindung betrifft neue Bis-o-aminophenole und o-Aminophenolcarbonsäuren sowie ein Verfahren zu deren Herstellung.

Bis-o-aminophenole und o-Aminophenolcarbonsäuren werden insbesondere zur Herstellung von hochtemperaturstabilen Polymeren, wie Polybenzoxazolen (PBO) bzw. deren Vorstufen, sowie zur Herstellung von Hydroxypolyimiden benötigt (siehe dazu beispielsweise EP-PS 0 264 678 und EP-PS 0 300 326). PBO-Vorstufen können in der Weise hergestellt werden, dass ein Dicarbonsäurechlorid mit einem Bis-o-aminophenol umgesetzt wird. Während aber wegen der Vielfalt industrieller Einsatzmöglichkeiten zahlreiche Dicarbonsäuren bzw. deren Chloride verfügbar sind, gibt es vergleichsweise wenige Bis-o-aminophenole. Außerdem beeinflusst die Art des eingesetzten Aminophenols in starkem Maße das Eigenschaftsspektrum des damit hergestellten Polymers. So werden nicht nur das thermische, elektrische und mechanische Verhalten, sondern auch die Löslichkeit und die Hydrolysestabilität sowie zahlreiche weitere Eigenschaften des Polymers durch das bei der Herstellung verwendete Aminophenol stark beeinflusst.

PBC-Vorstufen können in Form einer photosensitiven Zusammensetzung kostengünstig auf direktem Weg, d.h. ohne einen Hilfsresist, strukturiert werden. Im Vergleich mit anderen direkt photostrukturierbaren Dielektrika, wie Polyimid (PI) und Benzocyclobuten (BCB), bieten PBO-Vorstufen den Vorteil der positiven Strukturierbarkeit und der wässrig-alkalischen Entwicklung (siehe EP-PS 0 023 662 und EP-PS 0 264 678). Hierzu müssen die verwendeten PBO-Vorstufen bei der Belichtungswellenlänge weitgehend transparent und im - vorzugsweise metallionenfreien - Entwickler ausreichend löslich sein. Wie die Polyimide haben auch Polybenzoxazole den großen Vorteil, dass sie - im Vergleich zum cyclisierten Endprodukt - als gut lösliche Vorstufe auf ein Substrat aufgebracht und anschließend auf dem Substrat cyclisiert werden können, wobei die Löslichkeit und damit die Sensibilität gegenüber Lösemitteln und anderen Prozesschemikalien stark abnimmt.

Für den Einsatz von Polybenzoxazolen in der Mikroelektronik, insbesondere als Dielektrikum zwischen zwei Metallebenen, beispielweise bei Multi-Chip-Modulen sowie Speicher- und Logikchips, oder als Pufferschicht ("Buffercoat") zwischen dem Chip und seinem Gehäuse, sind gute elektrische, mechanische und thermische Eigenschaften erforderlich. Beim Einsatz von Polymeren der genannten Art als Dielektrikum zwischen metallischen Leiterbahnen ist es sehr wichtig, dass das Metall bei erhöhter Temperatur, d.h. bei einer Temperatur > 300°C, nicht durch das Dielektrikum diffundiert. Viele Metalle, insbesondere Aluminium, das derzeit am meisten verwendete Metall, diffundieren aber bei hohen Temperaturen durch das Dielektrikum. Aus diesem Grunde wird das Metall mit einer Sperrschicht, beispielsweise aus Titannitrid oder einer Kombination von Titan und Titannitrid, versehen, welche die Diffusion des Metalls in das Dielektrikum verhindert. Die Verwendung einer zusätzlichen Schicht erfordert jedoch einen deutlich höheren Kosten- und auch Zeitaufwand.

Bislang mangelt es an geeigneten Bis-o-aminophenolen und o-Aminophenolcarbonsäuren zur Herstellung von Polymeren, welche die stark gestiegenen Anforderungen in der Mikroelektronik erfüllen. Vergleichbare Monomere sind beispielsweise aus EP-OS 0 110 420, EP-OS 0 317 942, EP-OS 0 905 123 und EP-OS 0 906 903 bekannt. Hinweise auf die Diffusion von Metallen in den daraus hergestellten Polymeren nach der Cyclisierung auf einem Substrat gibt es aber nicht (siehe: EP-PS 0 264 678 und EP-OS 0 317 942).

Aufgabe der Erfindung ist es, Bis-o-aminophenole und o-Aminophenolcarbonsäuren bereitzustellen, die zur Herstellung von Polymeren geeignet sind, welche die stark gestiegenen Anforderungen der Mikroelektronik erfüllen. Diese Monomere sollen insbesondere die Herstellung gut löslicher Polymer-Vorstufen ermöglichen, die nach der Cyclisierung auf einem Substrat Polybenzoxazole mit hoher Temperaturstabilität und vor allem deutlich reduzierter Metalldiffusion ergeben.

Dies wird erfindungsgemäß durch Bis-o-aminophenole und o-Aminophenolcarbonsäuren folgender Struktur erreicht: 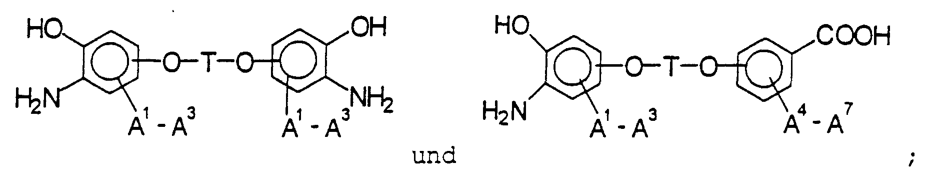 dabei gilt Folgendes:
A¹ bis A⁷ sind - unabhängig voneinander - H, F, CH₃, CF₃, OCH₃ oder OCF₃;
T ist einer der folgenden Reste: 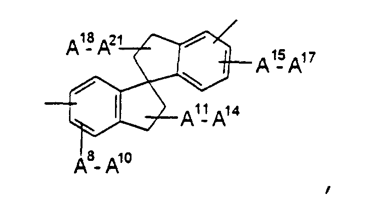 wobei A⁸ bis A²¹ - unabhängig voneinander - H, F, CH₃, CF₃, OCH₃ oder OCF₃ sind; 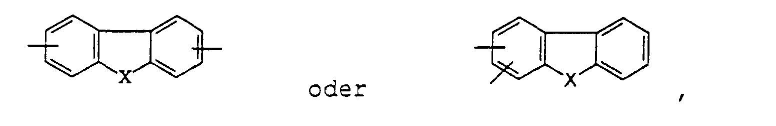 wobei X die Bedeutung CH₂, CF₂, C(CH₃)₂, C(CF₃)₂, C(OCH₃)₂, C (OCF₃)₂, C (CH₃) (C₆H₅), C (C₆H₅)₂, O, N-H, N-CH₃ oder N-C₆H₅ hat; 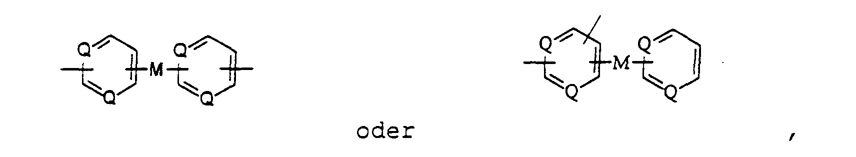 mit M = 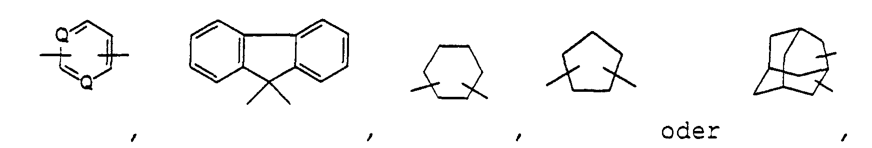
wobei Q jeweils C-H, C-F, C-CH₃, C-CF₃, C-OCH₃, C-OCF₃ oder N ist;
T und M können - unabhängig voneinander - auch einer der folgenden Reste sein: 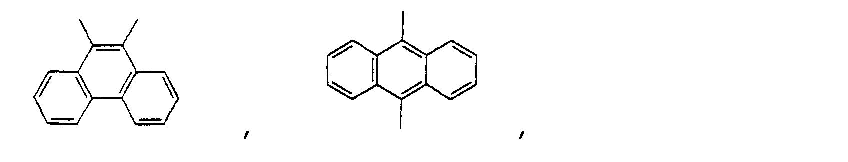 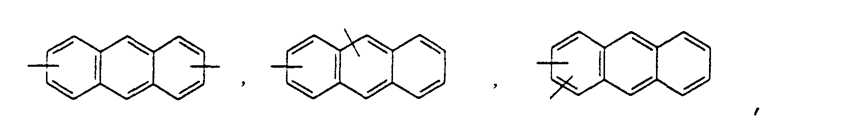 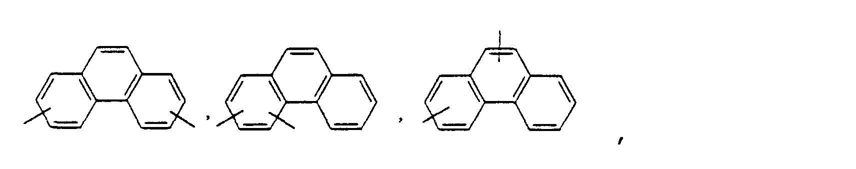 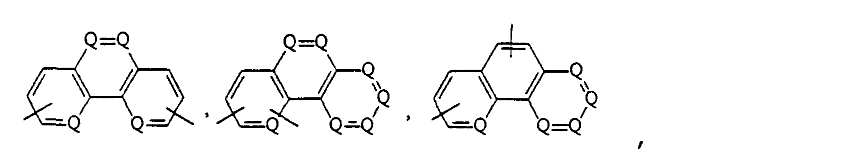 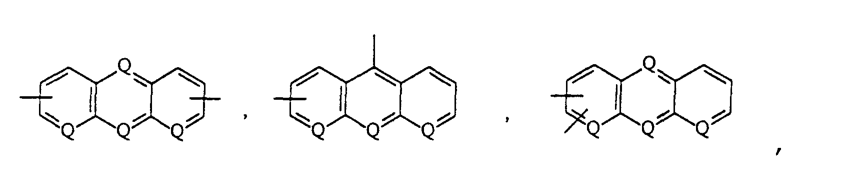 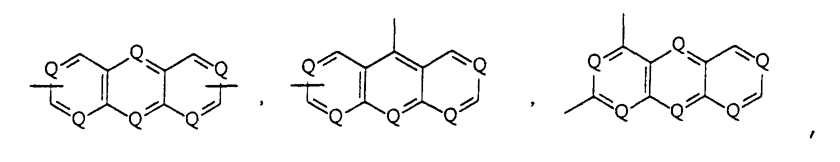 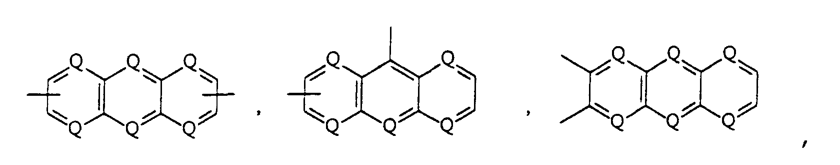
wobei Q wie vorstehend definiert ist, mindestens ein Q die Bedeutung N hat und maximal zwei N-Atome pro Ring vorhanden sein dürfen.

Die Charakterisierung "A¹-A³", "A⁴-A⁷", "A⁸-A¹⁰", "A¹¹-A¹⁴", "A¹⁵-A¹⁷" und "A¹⁸-A²¹" in den Strukturformeln bedeutet, dass die Phenylgruppen bzw. die cyclischen Gruppierungen Reste A¹, A² und A³ bzw. A⁴, A⁵, A⁶ und A⁷ bzw. A⁸, A⁹ und A¹⁰ bzw. A¹¹, A¹², A¹³ und A¹⁴ bzw. A¹⁵, A¹⁶ und A¹⁷ bzw. A¹⁸, A¹⁹, A²⁰ und A²¹ aufweisen.

Bei den neuen Verbindungen ist offensichtlich die spezielle Struktur für die gute Löslichkeit und die gute Sperrwirkung gegen Metalldiffusion der damit hergestellten Polymer-Vorstufen verantwortlich. Dielektrika, die aus derartigen Polybenzoxazol-Vorstufen hergestellt werden, zeigen insbesondere eine deutlich verringerte Aluminiumdiffusion. Dadurch wird eine zusätzliche Sperrschicht überflüssig.

Die Bis-o-aminophenole und o-Aminophenolcarbonsäuren werden in der Weise hergestellt, dass zunächst eine Dihydroxyverbindung (1) mit einem halogenierten o-Nitro-phenol-Derivat (2) umgesetzt wird. Die Umsetzung erfolgt in einem Lösemittel bei einer Temperatur von 50 bis 150°C in Gegenwart einer Base.

Bei der Herstellung von Bis-o-aminophenolen werden die Edukte (1) und (2) im Molverhältnis von etwa 1:2 eingesetzt, wobei die Umsetzung folgendermaßen verläuft:

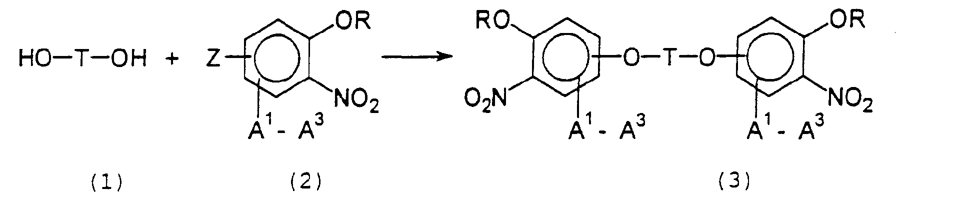

Bei der Herstellung von o-Aminophenolcarbonsäuren werden die Edukte (1) und (2) etwa im äquimolaren Verhältnis eingesetzt, wobei die Umsetzung folgendermaßen verläuft:

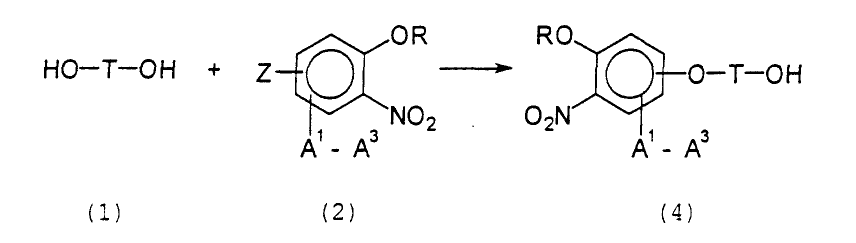

Das Reaktionsprodukt (4) wird dann mit einem halogenierten Benzolcarbonsäureester (5) umgesetzt, und zwar in einem Lösemittel bei einer Temperatur von 50 bis 150°C in Gegenwart einer Base. Dabei findet folgende Reaktion statt:

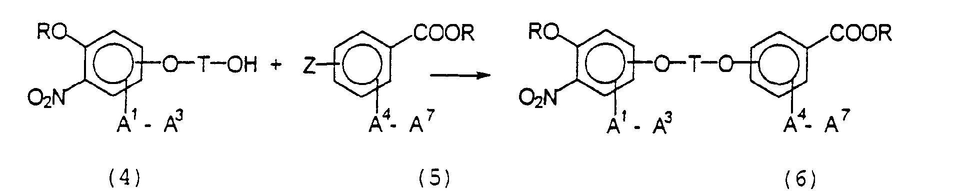

Die Reaktionsprodukte (3) und (6) werden dann einer Reduktion und einer Spaltung unterworfen, die Reaktionsprodukte (6) ferner einer Hydrolyse. Dabei laufen folgende Reaktionen ab, wobei als Endprodukte Bis-o-aminophenole (7) bzw. o-Aminophenolcarbonsäuren (8) gebildet werden:

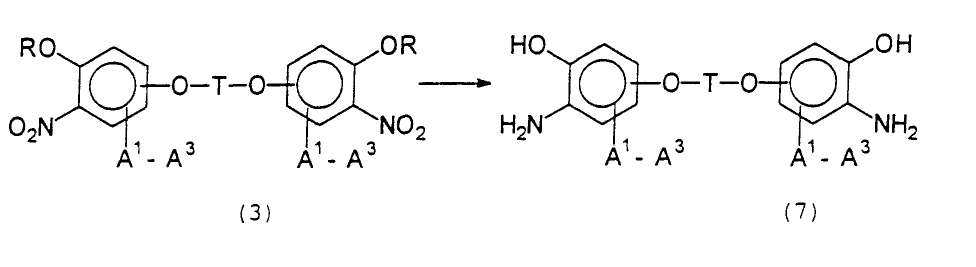

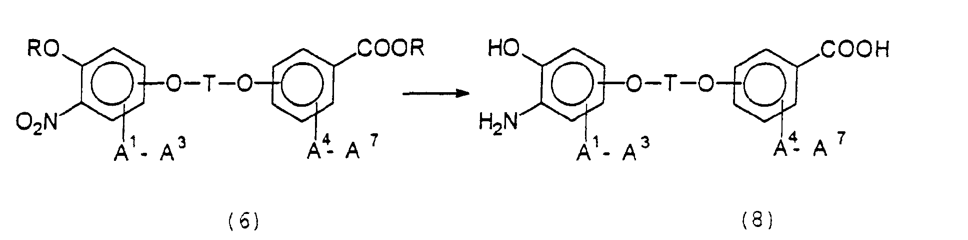

Die Reduktion der Nitroverbindungen (3) und (6) kann beispielsweise durch Hydrierung mit Wasserstoff an Palladium/ Aktivkohle (Pd/C) durchgeführt werden. Es sind aber prinzipiell alle Verfahren geeignet, die sich für die Reduktion der Nitrogruppe zur Aminogruppe eignen.

Zur Spaltung der RO-Gruppe bei den Verbindungen (3) und (6) kann beispielsweise Trifluoressigsäure oder Titantetrachlorid eingesetzt werden. Die Hydrolyse der Estergruppe bei den Verbindungen (6) kann beispielsweise mit Kaliumhydroxid erfolgen.

Die Reduktion und die Spaltung sowie die Hydrolyse können in getrennten Verfahrensschritten durchgeführt werden; die Reihenfolge der Verfahrensschritte ist dabei beliebig. Vorteilhaft erfolgen die Reaktionen, d.h. Reduktion und Spaltung bzw. Reduktion, Spaltung und Hydrolyse, jedoch gleichzeitig, d.h. zusammen. Dies geschieht vorzugsweise durch eine Hydrierung mit Wasserstoff an Pd/C.

Als Base dient vorzugsweise ein Carbonat oder Hydrogencarbonat eines Alkali- oder Erdalkalimetalls, wie Natriumcarbonat (Na₂CO₃) oder Kaliumcarbonat (K₂CO₃). Vorteilhaft kann aber auch eine organische Base mit einem tertiären N-Atom Verwendung finden, beispielsweise Triethylamin oder Pyridin.

In den vorstehend angeführten Formeln bedeutet Z ein Halogenatom, insbesondere Fluor. R ist einer der folgenden Reste: Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen. Vorzugsweise hat die Gruppe R folgende Bedeutung: Alkyl, Alkoxyalkyl, Phenyl oder Benzyl.

Die aus den Bis-o-aminophenolen und o-Aminophenolcarbonsäuren nach der Erfindung hergestellten Polymer-Vorstufen sind in vielen organischen Lösemitteln, wie Aceton, Ethyllactat, N-Methylpyrrolidon, Diethylenglykolmono- bzw. -diethylether, Cyclohexanon und γ-Butyrolacton, sowie in metallionenfreien wässrig-alkalischen Entwicklern gut löslich. Sie sind deshalb als Basispolymer für positiv photostrukturierbare und wässrig-alkalisch entwickelbare Dielektrika gut geeignet. Die Vorstufen können mittels Schleudertechnik problemlos auf Substrate, wie Siliziumwafer, aufgebracht werden, sie bilden gleichmäßige Filme und lassen sich auf dem Substrat gut cyclisieren. Ein besonderer Vorteil der aus den Bis-o-aminophenolen und o-Aminophenolcarbonsäuren hergestellten Vorstufen ist die im Vergleich zu bekannten Vorstufen deutlich reduzierte Metalldiffusion.

Anhand von Ausführungsbeispielen soll die Erfindung noch näher erläutert werden.

### Beispiel 1

### Herstellung von 9,9-Bis(4-[(3-benzyloxy-4-nitro)-phenoxy]phenyl}-fluoren

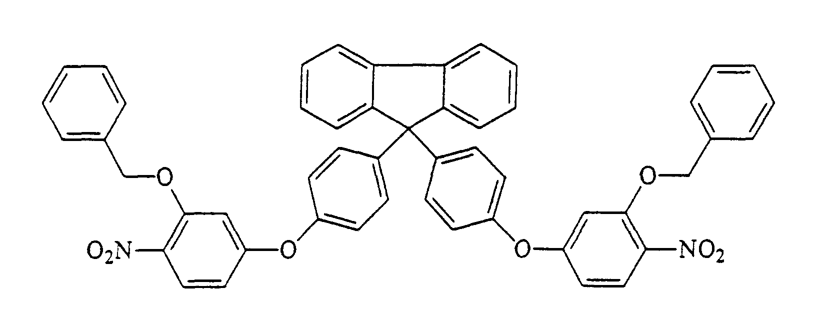

100,0 g 9,9-Bis(4-hydroxy-phenyl)-fluoren (0,285 mol) und 142,5 g 3-Fluor-6-nitro-benzyloxy-phenol (0,576 mol) werden in 1050 ml Dimethylformamid gelöst. Anschließend wird mit 200,0 g K₂CO₃ (1,447 mol) versetzt und unter ständigem Rühren 3 h auf 135°C erhitzt. Nach Beendigung der Reaktion wird auf Raumtemperatur abgekühlt und die Mischung unter Rühren in 2,5 1 Eiswasser gegossen. Der dabei ausfallende gelbe Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Zur Reinigung wird das Rohprodukt in der Siedehitze in 800 ml Tetrahydrofuran gelöst und mit 800 ml Petroleumbenzin (Siedebereich 60 bis 80°C) versetzt. Zum Kristallisieren wird 12 h bei 4°C gelagert, anschließend wird über einen Büchnertrichter abfiltriert und der weiße Feststoff im Vakuumschrank unter Stickstoff bei 50°C/50 mbar getrocknet; Ausbeute: 211,0 g.

Charakterisierung:
- Massenspektrum: Molekülpeak bei 804
- Elementaranalyse:

| | C | H | N |
|---|---|---|---|
| Theoretischer Wert (in %) | 76,1 | 4,5 | 3,5 |
| Gemessener Wert (in %) | 76,1 | 4,4 | 3,5 |

### Beispiel 2

### Herstellung von 9,9-Bis(4-[(4-amino-3-hydroxy)-phenoxy]phenyl}-fluoren

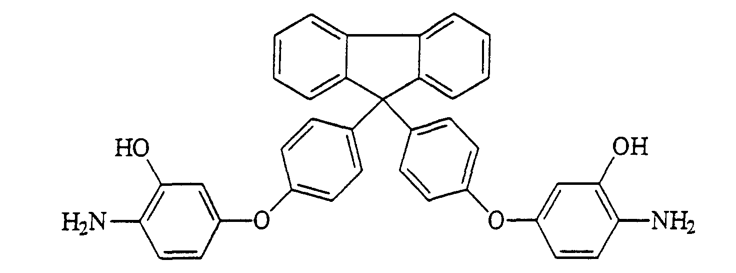

70,0 g des entsprechend Beispiel 1 hergestellten 9,9-Bis{4-[(3-benzyloxy-4-nitro)-phenoxy]-phenyl}-fluoren (0,087 mol) werden in 700 ml Tetrahydrofuran gelöst, die Lösung wird mit 7,0 g Palladium auf Aktivkohle versetzt und die Suspension in einem Autoklaven bei Raumtemperatur mit Wasserstoff bei einem Druck von 2 bar hydriert; die Reaktion ist nach 24 h beendet. Die Suspension wird dann über einen Büchnertrichter abfiltriert. Von der dabei erhaltenen roten Lösung wird mittels eines Rotationsverdampfers das Lösemittel vollständig entfernt.

Zur Reinigung wird das orangefarbene Rohprodukt in der Siedehitze in 500 ml Tetrahydrofuran gelöst und mit 150 ml Petroleumbenzin (Siedebereich 60 bis 80°C) versetzt. Zum Kristallisieren wird 12 h bei 4°C gelagert, anschließend wird über einen Büchnertrichter abfiltriert und der schwach gelbe Feststoff in einem Vakuumschrank unter Stickstoff bei 50°C/ 50 mbar getrocknet; Ausbeute: 47,2 g.

Charakterisierung:
- Massenspektrum: Molekülpeak bei 564
- Elementaranalyse:

| | C | H | N |
|---|---|---|---|
| Theoretischer Wert (in %) | 78,7 | 5,0 | 5,0 |
| Gemessener Wert (in %) | 78,6 | 5,1 | 5,1 |

### Beispiel 3

### Herstellung von 9-(4-[(3-Benzyloxy-4-nitro)-phenoxy]-phenyl}-9-(4-hydroxy-phenyl)-fluoren

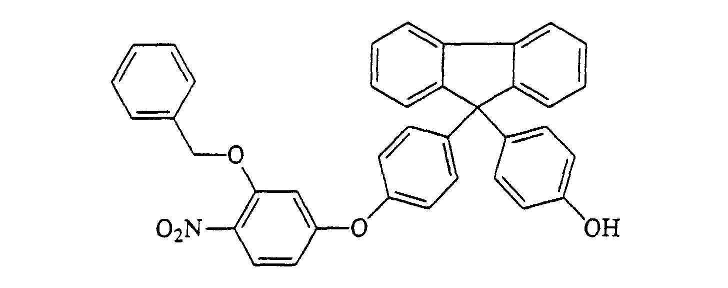

50,0 g 9,9-Bis(4-hydroxy-phenyl)-fluoren (0,143 mol) und 35,28 g 3-Fluor-6-nitro-benzyloxy-phenol (0,143 mol) werden in 550 ml Dimethylformamid gelöst. Anschließend wird mit 100,0 g K₂CO₃ (0,724 mol) versetzt und unter ständigem Rühren 5 h auf 120°C erhitzt. Nach Beendigung der Reaktion wird auf Raumtemperatur abgekühlt und die Mischung unter Rühren in 1,0 1 Eiswasser gegossen. Der dabei ausfallende gelbe Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 350 ml Tetrahydrofuran und 350 ml Petroleumbenzin; Ausbeute: 73,51 g.

Charakterisierung:
- Massenspektrum: Molekülpeak bei 577
- Elementaranalyse:

| | C | H | N |
|---|---|---|---|
| Theoretischer Wert (in %) | 79,0 | 4,7 | 2,4 |
| Gemessener Wert (in %) | 78,8 | 4,7 | 2,4 |

### Beispiel 4

### Herstellung von 9-(4-[(3-Benzyloxy-4-nitro)-phenoxy]-phenyl}-9-{4-[(2-trifluormethyl-4-benzylcarboxy)-phenoxy]-phenyl}-fluoren

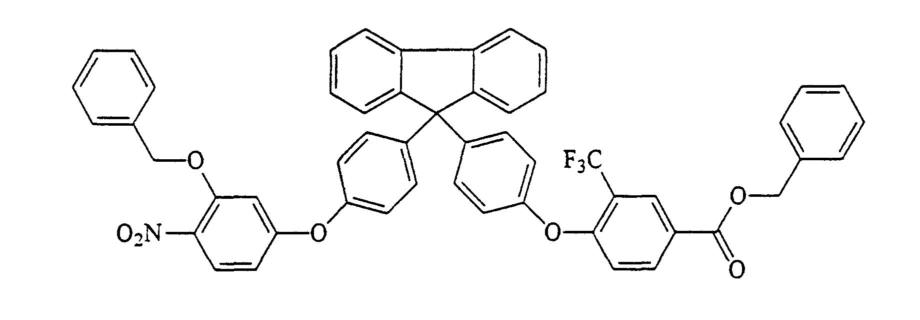

50,0 g des entsprechend Beispiel 3 hergestellten 9-(4-[(3-Benzyloxy-4-nitro)-phenoxy]-phenyl}-9-(4-hydroxy-phenyl)fluoren (0,0866 mol) und 25,83 g 4-Fluor-3-trifluormethylbenzoesäurebenzylester (0,0866 mol) werden in 400 ml Dimethylformamid gelöst. Anschließend wird mit 31,40 g K₂CO₃ (0,2272 mol) versetzt und unter ständigem Rühren 3 h auf 135°C erhitzt. Nach Beendigung der Reaktion wird auf Raumtemperatur abgekühlt, die Mischung über ein Faltenfilter abfitriert und in 1 l Wasser gegossen; dann wird 3 mal mit insgesamt 600 ml Essigester extrahiert. Die vereinigten organischen Phasen werden noch 2 mal mit Wasser gewaschen, und die erhaltene farblose Lösung wird am Rotationsverdampfer bis zur Trockene eingedampft; dabei wird ein weißer Feststoff erhalten.

Die Reinigung des Rohproduktes durch Umkristallisieren erfolgt entsprechend Beispiel 1 mit 350 ml Tetrahydrofuran und 350 ml Petroleumbenzin; Ausbeute: 68,64 g.

Charakterisierung:
- Massenspektrum: Molekülpeak bei 855
- Elementaranalyse:

| | C | H | N |
|---|---|---|---|
| Theoretischer Wert (in %) | 74,4 | 4, 2 | 1,6 |
| Gemessener Wert (in %) | 74,5 | 4,2 | 1,6 |

### Beispiel 5

### Herstellung von 9-{4-[(4-Amino-3-hydroxy) -phenoxy]-phenyl}-9-{4-[2-trifluormethyl-4-carboxy]-phenoxy)-phenyl}-fluoren

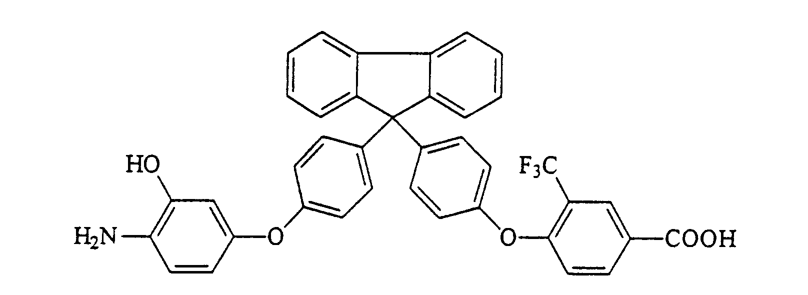

50,0 g des entsprechend Beispiel 4 hergestellten 9-{4-[(3-Benzyloxy-4-nitro)-phenoxy]-phenyl}-9-(4-[(2-trifluormethyl-4-benzylcarboxy)-phenoxy]-phenyl}-fluoren (0,058 mol) werden in 700 ml Tetrahydrofuran gelöst, die Lösung wird mit 5,0 g Palladium auf Aktivkohle versetzt und die Suspension in einem Autoklaven bei Raumtemperatur mit Wasserstoff bei einem Druck von 2 bar hydriert; die Reaktion ist nach 24 h beendet. Die Suspension wird dann über einen Büchnertrichter abfiltriert. Von der dabei erhaltenen hellbraunen Lösung wird mittels eines Rotationsverdampfers das Lösemittel vollständig entfernt.

Die Reinigung des Rohproduktes durch Umkristallisieren erfclgt entsprechend Beispiel 2 mit 350 ml Tetrahydrofuran und 100 ml Petroleumbenzin; Ausbeute: 35,2 g.

Charakterisierung:
- Massenspektrum: Molekülpeak bei 645
- Elementaranalyse:

| | C | H | N |
|---|---|---|---|
| Theoretischer Wert (in %) | 72,6 | 4,1 | 2,2 |
| Gemessener Wert (in %) | 72,7 | 4,1 | 2,1 |

### Beispiel 6

### Herstellung von 4,7-Bis[(3-benzyloxy-4-nitro)-phenoxy]-1,10-phenanthrolin

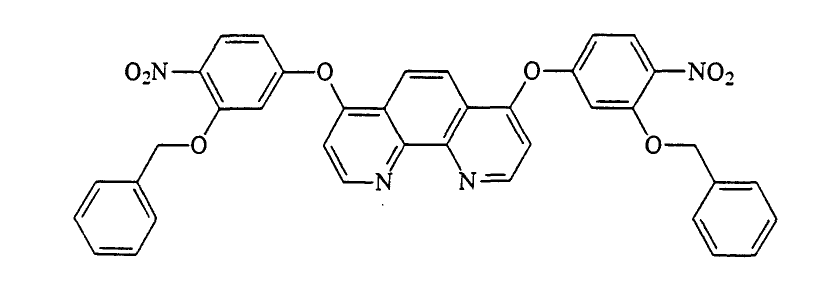

25,0 g 4,7-Dihydroxy-1,10-phenanthrolin (0,118 mol) und 59,8 g 3-Fluor-6-nitro-benzyloxy-phenol (0,241 mol) werden in 250 ml Dimethylsulfoxid gelöst. Anschließend wird mit 40,8 g K₂CO₃ (0,295 mol) versetzt und unter ständigem Rühren 6 h auf 120°C erhitzt. Nach Beendigung der Reaktion wird auf Raumtemperatur abgekühlt und die Mischung unter Rühren in 700 ml Wasser gegossen. Der dabei ausfallende gelbe Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 300 ml Tetrahydrofuran und 300 ml Petroleumbenzin; Ausbeute: 68,0 g.

Charakterisierung:
- Massenspektrum: Molekülpeak bei 666
- Elementaranalyse:

| | C | H | N |
|---|---|---|---|
| Theoretischer Wert (in %) | 68,5 | 3,9 | 8,4 |
| Gemessener Wert (in %) | 68,3 | 3,9 | 8,4 |

### Beispiel 7

### Herstellung von 4,7-Bis[(4-amino-3-hydroxy)-phenoxy]-1,10-phenanthrolin

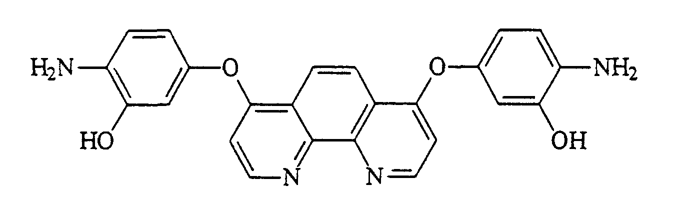

50,0 g des entsprechend Beispiel 6 hergestellten 4,7-Bis[(3-benzyloxy-4-nitro)-phenoxy]-1,10-phenanthrolin (0,075 mol) werden in 700 ml Tetrahydrofuran gelöst, die Lösung wird mit 5 g Palladium auf Aktivkohle versetzt und die Suspension in einem Autoklaven bei Raumtemperatur mit Wasserstoff bei einem Druck von 2 bar hydriert; die Reaktion ist nach 24 h beendet.

Die Suspension wird dann über einen Büchnertrichter abfiltriert. Von der dabei erhaltenen rotbraunen Lösung wird mittels eines Rotationsverdampfers das Lösemittel vollständig entfernt.

Zur Reinigung wird das Rohprodukt aus Essigsäureethylester und Petroleumbenzin (Siedebereich 60 bis 80°C) umkristallisiert und der schwach braune Feststoff in einem Vakuumschrank unter Stickstoff bei 50°C/50 mbar getrocknet; Ausbeute: 29,8 g.

Charakterisierung:
- Massenspektrum: Molekülpeak bei 426
- Elementaranalyse:

| | C | H | N |
|---|---|---|---|
| Theoretischer Wert (in %) | 67,6 | 4,3 | 13,1 |
| Gemessener Wert (in %) | 67,6 | 4,4 | 13,0 |

### Beispiel 8

### Herstellung von 4-[(3-Benzyloxy-4-nitro)-phenoxy]-7-hydroxy-1,10-phenanthrolin

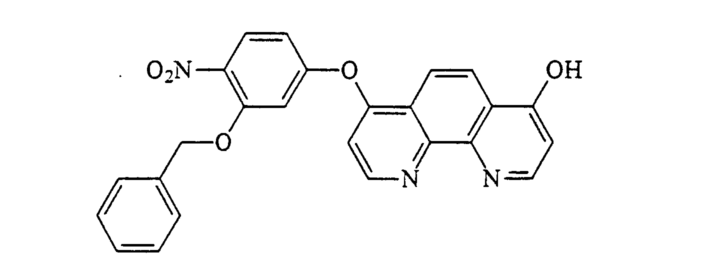

25,0 g 4,7-Dihydroxy-1,10-phenanthrolin (0,118 mol) und 29,2 g 3-Fluor-6-nitro-benzyloxy-phenol (0,118 mol) werden in 500 ml Dimethylsulfoxid gelöst. Anschließend wird mit 40,8 g K₂CO₃ (0,295 mol) versetzt und unter ständigem Rühren 12 h auf 110°C erhitzt. Nach Beendigung der Reaktion wird auf Raumtemperatur abgekühlt und die Mischung unter Rühren in 700 ml Wasser gegossen. Der dabei ausfallende Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 200 ml Tetrahydrofuran und 200 ml Petroleumbenzin; Ausbeute: 45,3 g.

Charakterisierung:
- Massenspektrum: Molekülpeak bei 439
- Elementaranalyse:

| | C | H | N |
|---|---|---|---|
| Theoretischer Wert (in %) | 68,3 | 3,9 | 9,6 |
| Gemessener Wert (in %) | 68,3 | 3,9 | 9,6 |

### Beispiel 9

### Herstellung von 4-[(3-Benzyloxy-4-nitro)-phenoxy]-7-[(4-benzylcarboxy)-phenoxy]-1,10-phenanthrolin

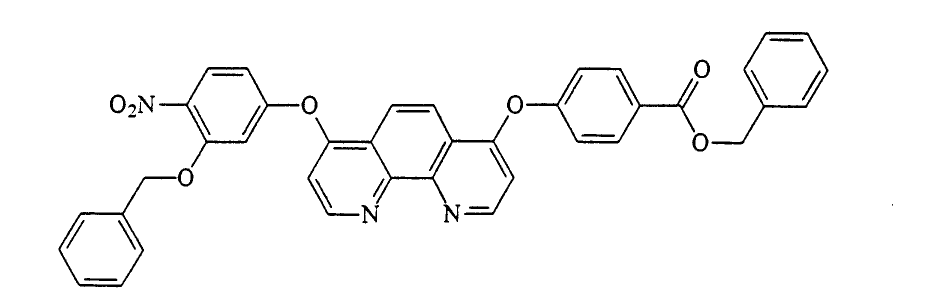

25,0 g des entsprechend Beispiel 8 hergestellten 4-[(3-Benzyloxy-4-nitro)-phenoxy]-7-hydroxy-1,10-phenanthrolin (0,057 mol) und 13,1 g 4-Fluor-benzoesäurebenzylester (0,057 mol) werden in 500 ml Dimethylsulfoxid gelöst. Anschließend wird mit 40,8 g K₂CO₃ (0,295 mol) versetzt und unter ständigem Rühren 8 h auf 135°C erhitzt. Nach Beendigung der Reaktion wird auf Raumtemperatur abgekühlt, die Mischung über ein Faltenfilter abfitriert und in 700 ml Wasser gegossen; dann wird 3 mal mit insgesamt 600 ml Essigester extrahiert. Die vereinigten organischen Phasen werden noch 2 mal mit Wasser gewaschen, und die erhaltene farblose Lösung wird am Rotationsverdampfer bis zur Trockene eingedampft; es wird ein weißer Feststoff erhalten.

Die Reinigung des Rohproduktes durch Umkristallisieren erfolgt entsprechend Beispiel 1 mit 200 ml Tetrahydrofuran und 200 ml Petroleumbenzin; Ausbeute: 35,7 g.

Charakterisierung:
- Massenspektrum: Molekülpeak bei 649
- Elementaranalyse:

| | C | H | N |
|---|---|---|---|
| Theoretischer Wert (in %) | 72,1 | 4,2 | 6,5 |
| Gemessener Wert (in %) | 72,1 | 4,3 | 6,6 |

### Beispiel 10

### Herstellung von 4-[(4-Amino-3-hydroxy)-phenoxy]-7-[(4-carboxy)-phenoxy]-1,10-phenanthrolin

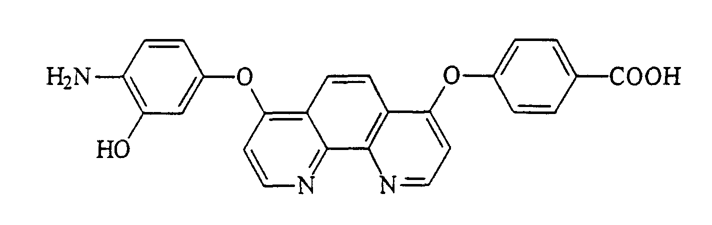

25,0 g des entsprechend Beispiel 9 hergestellten 4-[(3-Benzyloxy-4-nitro)-phenoxy]-7-[(4-benzylcarboxy)-phenoxy]-1,10-phenanthrolin (0,038 mol) werden in 400 ml Tetrahydrofuran gelöst, die Lösung wird mit 2,5 g Palladium auf Aktivkohle versetzt und die Suspension in einem Autoklaven bei Raumtemperatur mit Wasserstoff bei einem Druck von 2 bar hydriert; die Reaktion ist nach 24 h beendet. Die Suspension wird dann über einen Büchnertrichter abfiltriert. Von der dabei erhaltenen hellbraunen Lösung wird mittels eines Rotationsverdampfers das Lösemittel vollständig entfernt.

Zur Reinigung wird das Rohprodukt in der Siedehitze in 200 ml Tetrahydrofuran gelöst und mit 250 ml Petroleumbenzin (Siedebereich 60 bis 80°C) versetzt. Zum Kristallisieren wird 24 h bei 4°C gelagert, anschließend wird über einen Büchnertrichter abfiltriert und der schwach bräunliche Feststoff in einem Vakuumschrank unter Stickstoff bei 50°C/50 mbar getrocknet; Ausbeute: 15, 5 g.

Charakterisierung:
- Massenspektrum: Molekülpeak bei 439
- Elementaranalyse:

| | C | H | N |
|---|---|---|---|
| Theoretischer Wert (in %) | 68,3 | 3,9 | 9,6 |
| Gemessener Wert (in %) | 68,2 | 4,0 | 9,6 |

### Beispiel 11

### Herstellung von 6,6'-Bis[(3-benzyloxy-4-nitro)-phenoxy]-5,5'-dimethyl-3,3,3',3'-tetramethyl-1,1'-spiro-bis(indan)

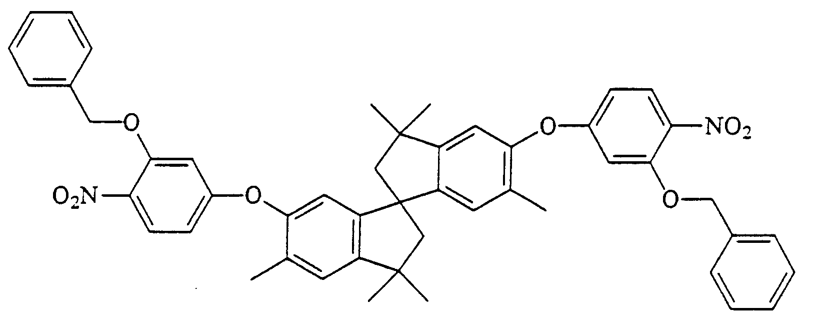

10,0 g 6,6'-Dihydroxy-5,5'-dimethyl-3,3,3',3'-tetramethyl-1,1'-spiro-bis(indan) (0,0297 mol) und 15,42 g 3-Fluor-6-nitro-benzyloxy-phenol (0,0624 mol) werden in 50 ml Dimethylformamid gelöst. Anschließend wird mit 10,26 g K₂CO₃ (0,0743 mol) versetzt und unter ständigem Rühren 3 h auf 130°C erhitzt. Nach Beendigung der Reaktion wird auf Raumtemperatur abgekühlt und die Mischung unter Rühren in 200 ml Wasser gegossen. Der dabei ausfallende Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 100 ml Tetrahydrofuran und 100 ml Petroleumbenzin; Ausbeute: 21,60 g.

Charakterisierung:
- Massenspektrum: Molekülpeak bei 790
- Elementaranalyse:

| | C | H | N |
|---|---|---|---|
| Theoretischer Wert (in %) | 74,4 | 5,9 | 3,5 |
| Gemessener Wert (in %) | 74, 4 | 5, 9 | 3, 4 |

### Beispiel 12

### Herstellung von 6,6'-Bis[(4-amino-3-hydroxy)-phenoxy]-5,5'-dimethyl-3,3,3',3'-tetramethyl-1,1'-spiro-bis(indan)

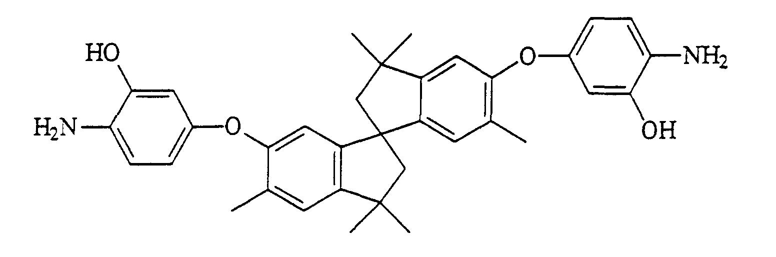

15,0 g des entsprechend Beispiel 11 hergestellten 6,6'-Bis-[(3-benzyloxy-4-nitro)-phenoxy]-5,5'-dimethyl-3,3,3',3'-tetramethyl-1,1'-spiro-bis (indan) (0,019 mol) werden in 150 ml Tetrahydrofuran gelöst, die Lösung wird mit 1,5 g Palladium auf Aktivkohle versetzt und die Suspension in einem Autoklaven bei Raumtemperatur mit Wasserstoff bei einem Druck von 2 bar hydriert; die Reaktion ist nach 24 h beendet. Die Suspension wird dann über einen Büchnertrichter abfiltriert. Von der dabei erhaltenen orangefarbenen Lösung wird mittels eines Rotationsverdampfers das Lösemittel vollständig entfernt.

Zur Reinigung wird das Rohprodukt in der Siedehitze in 100 ml Tetrahydrofuran gelöst und mit 250 ml Petroleumbenzin (Siedebereich 60 bis 80°C) versetzt. Zum Kristallisieren wird 24 h bei 4°C gelagert, anschließend wird über einen Büchnertrichter abfiltriert und der schwach bräunliche Feststoff in einem Vakuumschrank unter Stickstoff bei 50°C/50 mbar getrocknet; Ausbeute: 9,8 g.

Charakterisierung:
- Massenspektrum: Molekülpeak bei 550
- Elementaranalyse:

| | C | H | N |
|---|---|---|---|
| Theoretischer Wert (in %) | 76,3 | 7,0 | 5,1 |
| Gemessener Wert (in %) | 76,4 | 7, 1 | 5,1 |

### Beispiel 13

### Herstellung von 6-[(3-Benzyloxy-4-nitro)-phenoxy]-6'-hydroxy-5,5'-dimethyl-3,3,3',3'-tetramethyl-1,1'-spiro-bis(indan)

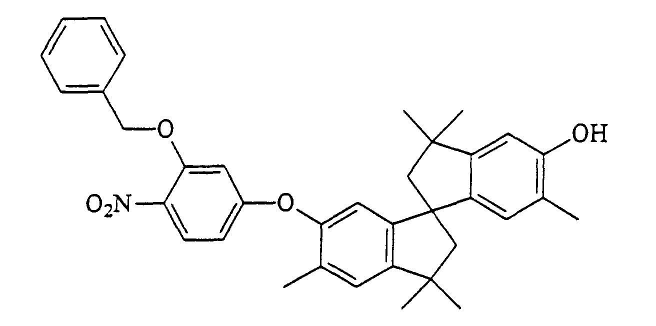

10,0 g 6,6'-Dihydroxy-5,5'-dimethyl-3,3,3',3'-tetramethyl-1,1'-spiro-bis(indan) (0,0297 mol) und 7,34 g 3-Fluor-6-nitro-benzyloxy-phenol (0,0297 mol) werden in 50 ml Dimethylformamid gelöst. Anschließend wird mit 10,26 g K₂CO₃ (0,0743 mol) versetzt und unter ständigem Rühren 5 h auf 120°C erhitzt. Nach Beendigung der Reaktion wird auf Raumtemperatur abgekühlt und die Mischung unter Rühren in 700 ml Wasser gegossen. Der dabei ausfallende Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 80 ml Tetrahydrofuran und 80 ml Petroleumbenzin; Ausbeute: 16,0 g.

Charakterisierung:
- Massenspektrum: Molekülpeak bei 563
- Elementaranalyse:

| | C | H | N |
|---|---|---|---|
| Theoretischer Wert (in %) | 76,7 | 6,6 | 2,5 |
| Gemessener Wert (in %) | 76,7 | 6,6 | 2,5 |

### Beispiel 14

### Herstellung von 6-[(3-Benzyloxy-4-nitro)-phenoxy]-6'-[(4-benzylcarboxy)-phenoxy]-5,5'-dimethyl-3,3,3',3'-tetramethyl-1,1'-spiro-bis (indan)

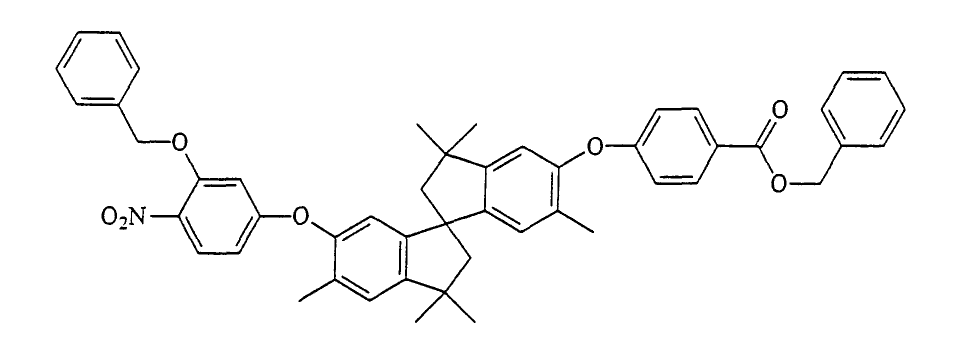

15,0 g des entsprechend Beispiel 13 hergestellten 6-[(3-Benzyloxy-4-nitro)-phenoxy]-6'-hydroxy-5,5'-dimethyl-3,3,3',3'-tetramethyl-1,1'-spiro-bis(indan) (0,0266 mol) und 6,13 g 4-Fluor-benzoesäurebenzylester (0,0266 mol) werden in 75 ml Dimethylformamid gelöst. Anschließend wird mit 4,95 g K₂CO₃ (0,0358 mol) versetzt und unter ständigem Rühren 5 h auf 130°C erhitzt. Nach Beendigung der Reaktion wird auf Raumtemperatur abgekühlt und die Mischung unter Rühren in 400 ml Wasser gegossen. Der dabei ausfallende Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 100 ml Tetrahydrofuran und 100 ml Petroleumbenzin; Ausbeute: 18,50 g.

Charakterisierung:
- Massenspektrum: Molekülpeak bei 773
- Elementaranalyse:

| | C | H | N |
|---|---|---|---|
| Theoretischer Wert (in %) | 77,6 | 6, 1 | 1,8 |
| Gemessener Wert (in %) | 77, 6 | 6, 0 | 1, 9 |

### Beispiel 15

### Herstellung von 6-[(4-Amino-3-hydroxy)-phenoxy]-6'-[(4-carboxy)-phenoxy]- 5,5'-dimethyl-3,3,3',3'-tetramethyl-1,1'-spiro-bis(indan)

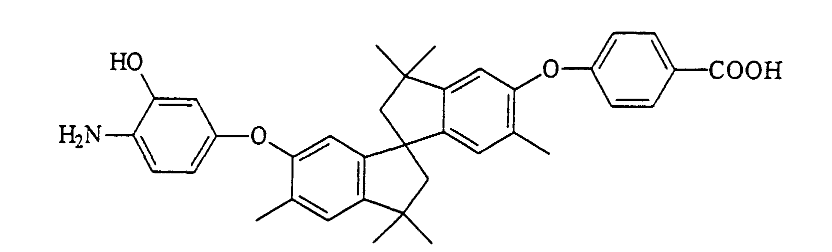

15,0 g des entsprechend Beispiel 14 hergestellten 6-[(3-Benzyloxy-4-nitro)-phenoxy]-6'-[(4-benzylcarboxy)-phenoxy]-5,5'-dimethyl-3,3,3',3'-tetramethyl-1,1'-spiro-bis (indan) (0,019 mol) werden in 200 ml Tetrahydrofuran gelöst, die Lösung wird mit 1,5 g Palladium auf Aktivkohle versetzt und die Suspension in einem Autoklaven bei Raumtemperatur mit Wasserstoff bei einem Druck von 2 bar hydriert; die Reaktion ist nach 24 h beendet. Die Suspension wird dann über einen Büchnertrichter abfiltriert. Von der dabei erhaltenen Lösung wird mittels eines Rotationsverdampfers das Lösemittel vollständig entfernt.

Die Reinigung des Rohproduktes durch Umkristallisieren erfolgt entsprechend Beispiel 2 mit 150 ml Tetrahydrofuran und 50 ml Petroleumbenzin; Ausbeute: 10,5 g.

Charakterisierung:
- Massenspektrum: Molekülpeak bei 563
- Elementaranalyse:

| | C | H | N |
|---|---|---|---|
| Theoretischer Wert (in %) | 76,7 | 6, 6 | 2,5 |
| Gemessener Wert (in %) | 76,5 | 6,7 | 2,5 |

### Beispiel 16

### Herstellung von 9, 10-Bis{4-[(3-benzyloxy-4-nitro)-phenoxy]-phenyl}-anthracen

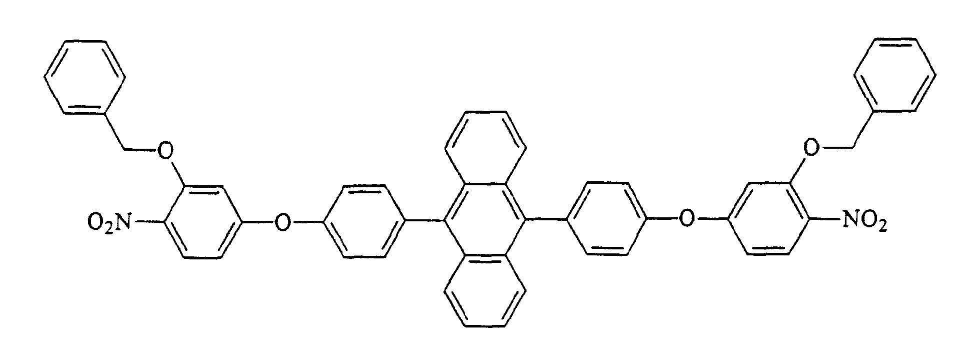

10,0 g 9,10-Bis(4-hydroxy-phenyl)-anthracen (0,0276 mol) und 14,0 g 3-Fluor-6-nitro-benzyloxy-phenol (0,0566 mol) werden in 300 ml Dimethylsulfoxid gelöst. Anschließend wird mit 9,53 g K₂CO₃ (0,069 mol) versetzt und unter ständigem Rühren 5 h auf 130°C erhitzt. Nach Beendigung der Reaktion wird auf Raumtemperatur abgekühlt und die Mischung unter Rühren in 600 ml Wasser gegossen. Der dabei ausfallende Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 100 ml Tetrahydrofuran und 100 ml Petroleumbenzin; Ausbeute: 21,73 g.

Charakterisierung:
- Massenspektrum: Molekülpeak bei 816
- Elementaranalyse:

| | C | H | N |
|---|---|---|---|
| Theoretischer Wert (in %) | 76,5 | 4,4 | 3,4 |
| Gemessener Wert (in %) | 76,4 | 4,4 | 3,4 |

### Beispiel 17

### Herstellung von 9, 10-Bis{4-[(4-amino-3-hydroxy)-phenoxy]-phenyl}-anthracen

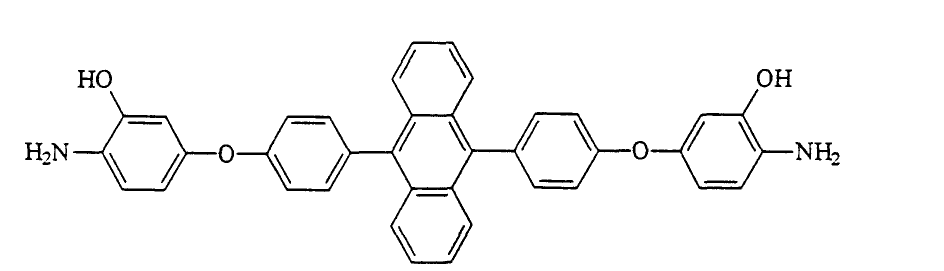

10,0 g des entsprechend Beispiel 16 hergestellten 9,10-Bis(4-[(3-benzyloxy-4-nitro)-phenoxy]-phenyl}-anthracen (0,012 mol) werden in 400 ml einer Mischung aus Tetrahydrofuran und Essigsäureethylester (Volumenverhältnis 1:1) gelöst, die Lösung wird mit 1,0 g Palladium auf Aktivkohle versetzt und die Suspension in einem Autoklaven bei Raumtemperatur mit Wasserstoff bei einem Druck von 2 bar hydriert; die Reaktion ist nach 24 h beendet. Die Suspension wird dann über einen Büchnertrichter abfiltriert. Von der dabei erhaltenen Lösung wird mittels eines Rotationsverdampfers das Lösemittel vollständig entfernt.

Die Reinigung des Rohproduktes durch Umkristallisieren erfolgt entsprechend Beispiel 2 mit 100 ml Tetrahydrofuran und 30 ml Petroleumbenzin; Ausbeute: 6,17 g.

Charakterisierung:
- Massenspektrum: Molekülpeak bei 576.
- Elementaranalyse:

| | C | H | N |
|---|---|---|---|
| Theoretischer Wert (in %) | 79,1 | 4,9 | 4,9 |
| Gemessener Wert (in %) | 79,1 | 5,0 | 5,0 |

### Beispiel 18

### Herstellung von 9-{4-[(3-Benzyloxy-4-nitro)-phenoxy]-phenyl}-10-(4-hydroxy-phenyl)-anthracen

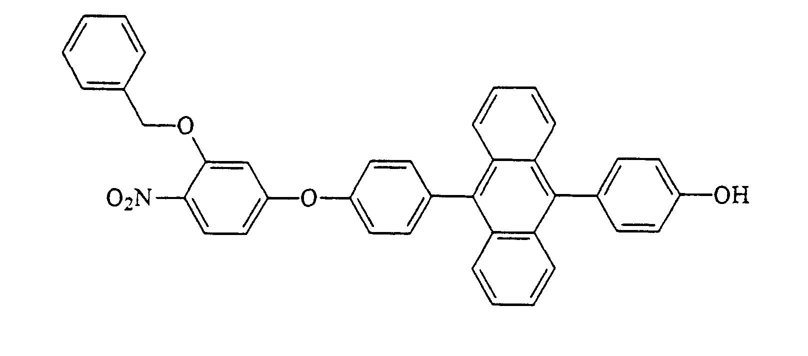

10,0 g 9,10-Bis(4-hydroxy-phenyl)-anthracen (0,0276 mol) und 6,82 g 3-Fluor-6-nitro-benzyloxy-phenol (0,0276 mol) werden in 300 ml Dimethylsulfoxid gelöst. Anschließend wird mit 9,53 g K₂CO₃ (0,069 mol) versetzt und unter ständigem Rühren 12 h auf 115°C erhitzt. Nach Beendigung der Reaktion wird auf Raumtemperatur abgekühlt und die Mischung unter Rühren in 600 ml Wasser gegossen. Der dabei ausfallende Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 100 ml Tetrahydrofuran und 100 ml Petroleumbenzin; Ausbeute: 15,80 g.

Charakterisierung:
- Massenspektrum: Molekülpeak bei 589
- Elementaranalyse:

| | C | H | N |
|---|---|---|---|
| Theoretischer Wert (in %) | 79,4 | 4,6 | 2,4 |
| Gemessener Wert (in %) | 79,4 | 4,6 | 2,4 |

### Beispiel 19

### Herstellung von 9-(4-[(3-Benzyloxy-4-nitro)-phenoxy]-phenyl}-l0-(4-[(4-benzylcarboxy)-phenoxy]-phenyl}-anthracen

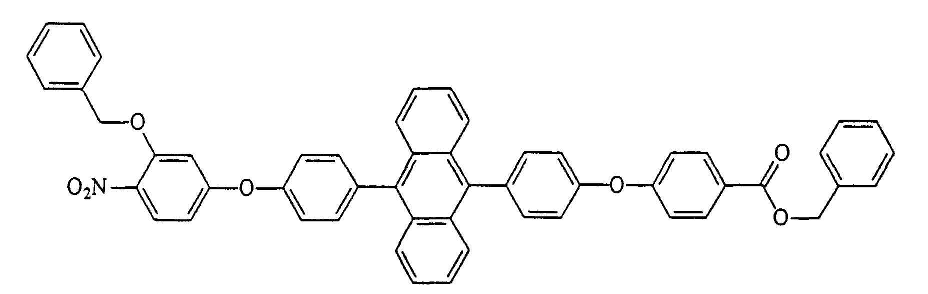

15,0 g des entsprechend Beispiel 18 hergestellten 9-{4-[(3-Benzyloxy-4-nitro)-phenoxy]-phenyl}-10-(4-hydroxy-phenyl)-anthracen (0,0254 mol) und 6,82 g 4-Fluor-benzoesäurebenzyl-ester (0,0296 mol) werden in 300 ml Dimethylsulfoxid gelöst. Anschließend wird mit 4,49 g K₂CO₃ (0,0325 mol) versetzt und unter ständigem Rühren 2 h auf 140°C erhitzt. Nach Beendigung der Reaktion wird auf Raumtemperatur abgekühlt und die Mischung unter Rühren in 600 ml Wasser gegossen. Der dabei ausfallende Feststoff wird über einen Büchnertrichter abfiltriert, je einmal mit verdünntem Eisessig und Wasser gewaschen und anschließend in einem Vakuumschrank unter Stickstoff bei 50°C/50 mbar getrocknet.

Die Reinigung des Rohproduktes durch Umkristallisieren erfolgt entsprechend Beispiel 1 mit 100 ml Tetrahydrofuran und 100 ml Petroleumbenzin; Ausbeute: 17,76 g.

Charakterisierung:
- Massenspektrum: Molekülpeak bei 799
- Elementaranalyse:

| | C | H | N |
|---|---|---|---|
| Theoretischer Wert (in %) | 79,6 | 4,7 | 1, 8 |
| Gemessener Wert (in %) | 79, 4 | 4, 6 | 1,7 |

### Beispiel 20

### Herstellung von 9-(4-[(4-Amino-3-hydroxy)-phenoxy]-phenyl}-10-(4-[(4-carboxy)-phenoxy]-phenyl}-anthracen

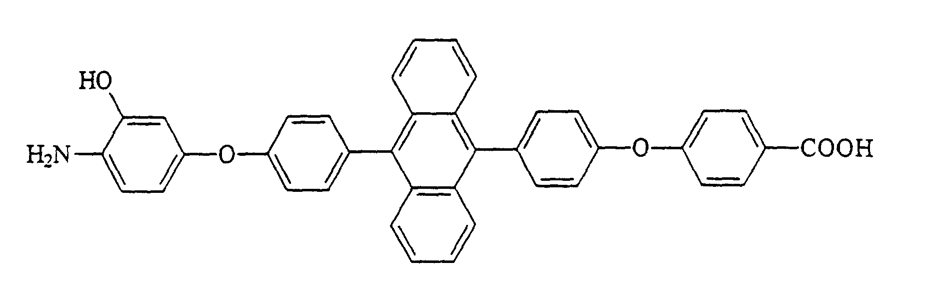

15,0 g des entsprechend Beispiel 19 hergestellten 9-{4-[(3-Benzyloxy-4-nitro)-phenoxy]-phenyl}-10-{4-[(4-benzylcarboxy)-phenoxy]-phenyl}-anthracen (0,0188 mol) werden in 600 ml einer Mischung aus Tetrahydrofuran und Essigsäureethylester (Volumenverhältnis 1:1) gelöst, die Lösung wird mit 1,5 g Palladium auf Aktivkohle versetzt und die Suspension in einem Autoklaven bei Raumtemperatur mit Wasserstoff bei einem Druck von 2 bar hydriert; die Reaktion ist nach 24 h beendet. Die Suspension wird dann über einen Büchnertrichter abfiltriert. Von der dabei erhaltenen Lösung wird mittels eines Rotationsverdampfers das Lösemittel vollständig entfernt.

Die Reinigung des Rohproduktes durch Umkristallisieren erfolgt entsprechend Beispiel 2 mit 100 ml Tetrahydrofuran und 30 ml Petroleumbenzin; Ausbeute: 10,91 g.

Charakterisierung:
- Massenspektrum: Molekülpeak bei 589.
- Elementaranalyse:

| | C | H | N |
|---|---|---|---|
| Theoretischer Wert (in %) | 79,4 | 4,6 | 2,4 |
| Gemessener Wert (in %) | 79,4 | 4,6 | 2,4 |

## Patentansprüche

1. Bis-o-aminophenole und o-Aminophenolcarbonsäuren der Struktur 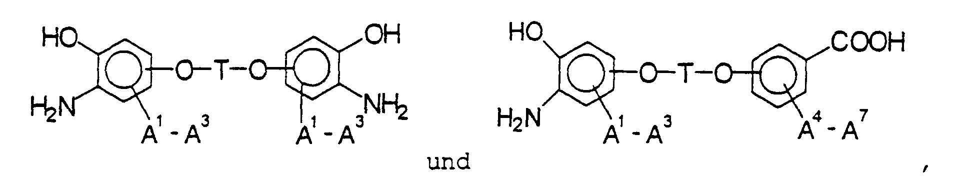 wobei Folgendes gilt:
A¹ bis A⁷ sind - unabhängig voneinander - H, F, CH₃, CF₃, OCH₃ oder OCF₃;
T ist einer der folgenden Reste: 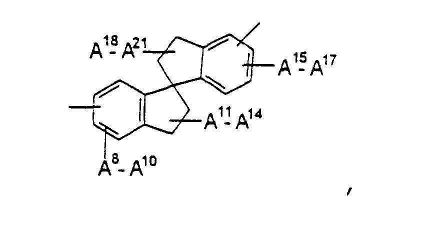 wobei A⁹ bis A²¹ - unabhängig voneinander - H, F, CH₃, CF₃, OCH₃ oder OCF₃ sind; 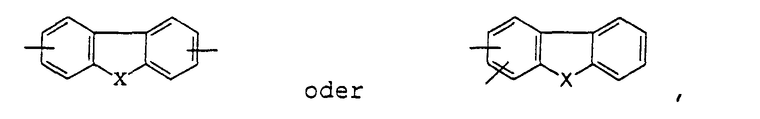 wobei X die Bedeutung CH₂, CF₂, C(CH₃)₂, C(CF₃)₂, C(OCH₃)₂, C(OCF₃)₂, C(CH₃)(C₆H₅), C(C₆H₅)₂, O, N-H, N-CH₃ oder N-C₆H₅ hat; 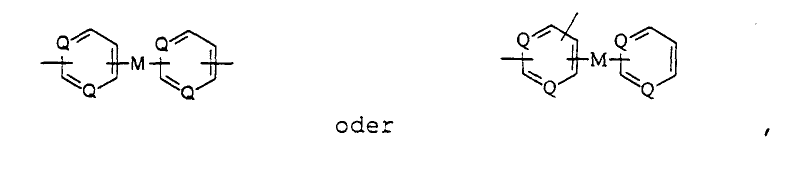 mit M = 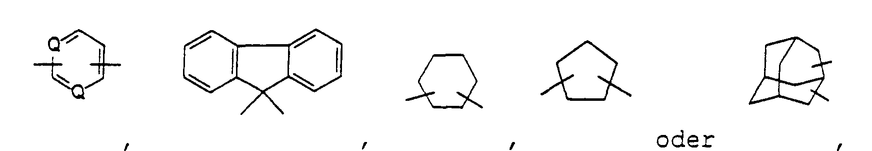
wobei Q jeweils C-H, C-F, C-CH₃, C-CF₃, C-OCH₃, C-OCF₃ oder N ist;
T und M können - unabhängig voneinander - auch einer der folgenden Reste sein: 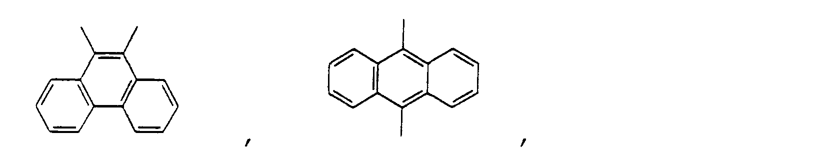 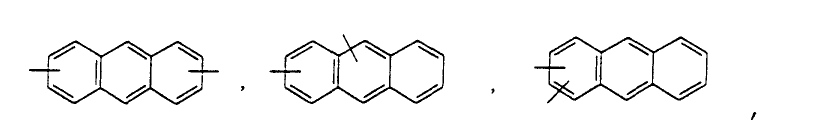 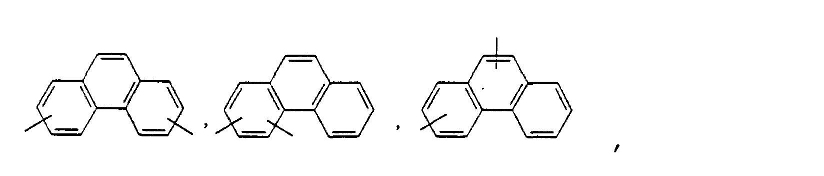 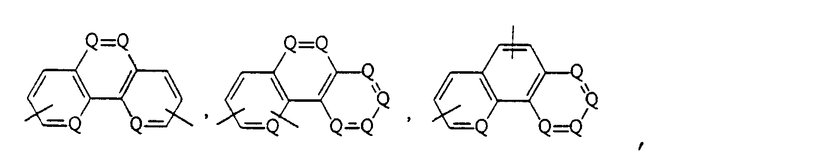 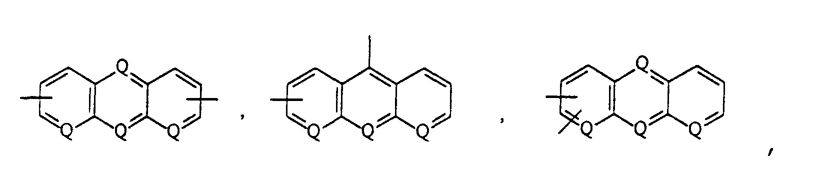 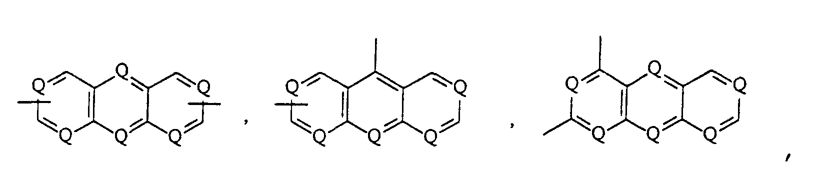 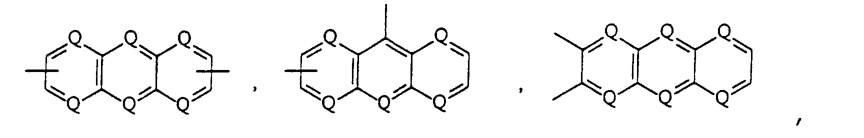 wobei Q wie vorstehend definiert ist, mindestens ein Q die Bedeutung N hat und maximal zwei N-Atome pro Ring vorhanden sein dürfen.

2. Verfahren zur Herstellung von Bis-o-aminophenolen nach Anspruch 1, **dadurch gekennzeichnet**, dass eine Dihydroxyverbindung mit einem halogenierten o-Nitro-phenol-Derivat im Molverhältnis von etwa 1:2 in einem Lösemittel bei einer Temperatur von 50 bis 150°C in Gegenwart einer Base zu einer Dinitroverbindung umgesetzt wird: 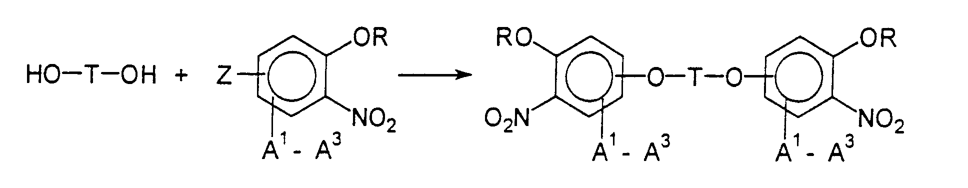 wobei Z ein Halogenatom ist, A¹ bis A³ und T die angegebene Bedeutung haben und R einer der folgenden Reste ist:
Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen,
und dass die Dinitroverbindung reduziert wird und die Gruppen R abgespalten werden:

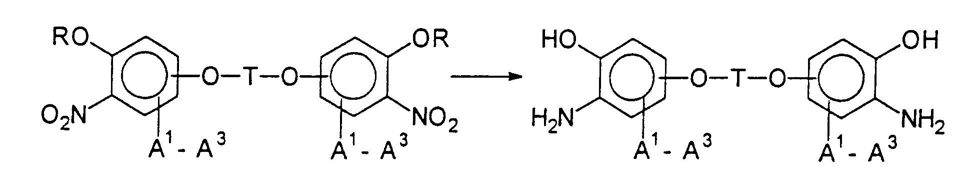

3. Verfahren zur Herstellung o-Aminophenolcarbonsäuren nach Anspruch 1, **dadurch gekennzeichnet, dass** eine Dihydroxyverbindung mit einem halogenierten o-Nitro-phenol-Derivat etwa im äquimolaren Verhältnis in einem Lösemittel bei einer Temperatur von 50 bis 150°C in Gegenwart einer Base zu einer Nitroverbindung umgesetzt wird: 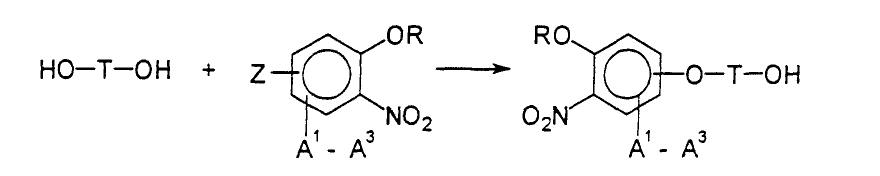 wobei Z ein Halogenatom ist, A¹ bis A³ und T die angegebene Bedeutung haben und R einer der folgenden Reste ist:
Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen,
dass die Nitroverbindung mit einem halogenierten Benzolcarbonsäureester in einem Lösemittel bei einer Temperatur von 50 bis 150°C in Gegenwart einer Base zu einer estergruppenhaltigen Nitroverbindung umgesetzt wird: 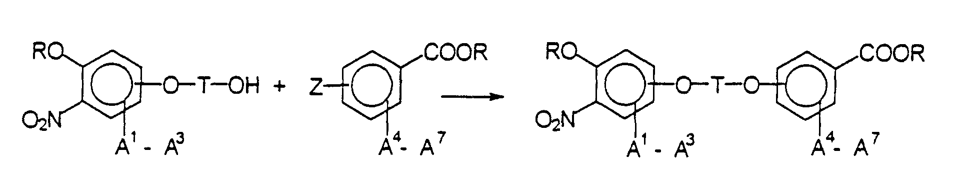
wobei Z, A¹ bis A³, A⁴ bis A⁷, T und R die angegebene Bedeutung haben,
und dass die Nitroverbindung reduziert sowie die Gruppe R abgespalten und die Estergruppe hydrolysiert wird:

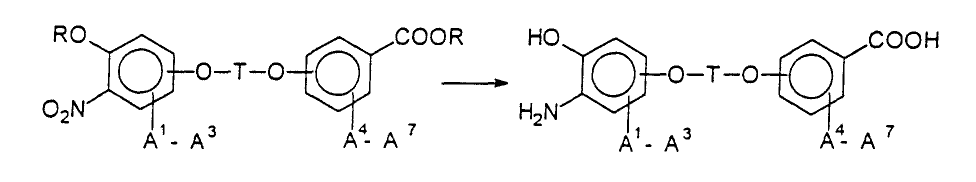

4. Verfahren nach Anspruch 2 oder 3, **dadurch gekennzeichnet, dass** als Base ein Carbonat oder Hydrogencarbonat eines Alkali- oder Erdalkalimetalls eingesetzt wird.

5. Verfahren nach Anspruch 2 oder 3, **dadurch gekennzeichnet, dass** eine organische Base mit einem tertiären N-Atom eingesetzt wird.

6. Verfahren nach einem der Ansprüche 2 bis 5, **dadurch gekennzeichnet**, dass die Reduktion und die Abspaltung der Gruppe(n) R sowie die Hydrolyse durch Wasserstoff erfolgt und mit Pd/C katalysiert wird.
